# EUROPEAN PATENT APPLICATION

(11) **EP 2 963 419 A1**
(43) Date of publication of application: **06.01.2016**
(21) Application number: 14756675.6
(22) Date of filing: 18.02.2014
(51) Int. Cl.: G01N 33/531, G01N 33/545, G01N 33/72

(54) **METHOD FOR PREVENTING DETERIORATION OF UNSENSITIZED LATEX REAGENT**

(30) Priority: 01.03.2013 JP 2013040337
(71) Applicant: Fujirebio Inc., Tokyo 163-0410 (JP)
(72) Inventor: TETSUMOTO, Toru, Tokyo 163-0410 (JP); HIRATA, Minoru, Tokyo 163-0410 (JP)
(74) Representative: Keirstead, Tanis Evelyne
(86) International application number: PCT/JP2014/053686
(87) International publication number: WO 2014/132833

(57) **Abstract**

Disclosed is novel means for favorably maintaining the dispersed state of a latex reagent in an immunoassay kit and preventing occurrence of coagulation/sedimentation due to temperature changes so that the reagent is protected from deterioration in the measurement performance, which means is also applicable to an immunoagglutination assay kit containing a latex reagent aliquoted in small amounts. The immunoassay reagent of the present invention comprises unsensitized latex particles and trimethylglycine in a solvent. The concentration of trimethylglycine in the reagent is preferably 5 to 10 w/v%. The reagent can be favorably used as an immunoassay reagent for, e.g., hemoglobin A1c.

SEQUENCE LISTING

<110>  TFB, INC.

<120>  Method for preventing deterioration of unsensitized latex reagent

<130>  PF505-PCT

<160>  1

<170>  PatentIn version 3.5

<210>  1
<211>  5
<212>  PRT
<213>  Homo sapiens

<400>  1

Val His Leu Thr Pro
1               5

## Description

### TECHNICAL FIELD

The present invention relates to a method for preventing deterioration of the performance of an immunoassay reagent, which reagent uses unsensitized latex.

### BACKGROUND ART

In general, in the so-called latex reagent in immunoassay kits, an antibody specifically reactive with the substance to be measured, or an antigenic substance reactive with the antibody to be measured, is immobilized beforehand on the surface of latex to be carried thereon. In order to increase shelf stability of the latex reagent, a sugar and/or a surfactant is/are added to the reagent, and freeze-drying is attempted (Patent Documents 1 and 2). The latex reagent could be stably stored by such a method. However, in such a method based on freeze-drying, reconstitution treatment of a latex solution is required before use, which is inconvenient for users.

Taking the convenience for users into account, a liquid latex reagent that can be used as it is, is desirable. A liquid latex reagent that does not require the reconstitution treatment before use generally contains a sugar, surfactant, and/or the like for prevention of deterioration due to freezing. However, the problem of peeling off of the immobilized antigen or antibody protein from the latex surface has not been completely solved, and there is no latex reagent which is free from the problem caused by freezing.

In cases of immunoassay reagents for hemoglobin A1c using unsensitized latex, prevention of freezing of the latex solution has been carried out mainly by thorough control of the temperature during storage and transportation. Since the volume of the latex reagent has been not less than 20 mL, deterioration of the reagent due to freezing has not been substantially problematic in the past.

However, in recent years, attempts are being made in general hospitals to collect blood and perform a blood test during the examination waiting time before the examination, thereby reflecting the test results in the examination. Reagents used in such situations are those for measurement using special compact devices, and such reagents are also aliquoted beforehand into cartridges such that each cartridge contains a reagent in an amount suitable for single test. The amount of the reagent is about 0.1 mL. Since data of a calibration curve prepared with standard samples are memorized in advance, changes in reactivity of the reagent lead to changes in the measured values. Thus, stability of the performance of the reagent is more strictly required than ever before. A reagent in such a small amount is more likely to be influenced by temperature changes during transportation, and the performance of the reagent is sometimes deteriorated. For example, in cases where supercooling of a product occurs due to a decrease in the temperature during refrigerated transportation, the reagent aliquoted in small amounts is highly likely to be frozen in a short time. Indeed, there are actual cases where, in an immunoagglutination assay kit for hemoglobin A1c using unsensitized latex, a latex reagent aliquoted in small amounts are supercooled during refrigerated transportation to be frozen, and as a result coagulation/sedimentation of latex occurs and hence the reagent becomes unusable. Thus, in recent years, protection of latex reagents from deterioration due to freezing has been an urgent issue.

Patent Document 2 describes antifreezing agents to be added when freeze-drying of sensitized latex is carried out, and, as examples of the antifreezing agents, high-molecular-weight polysaccharides, PVP, and surfactants such as Tween 20 are mentioned in the document.

Patent Document 3 mentions that an agglutination-measuring reagent and a measurement method which promote agglutination based on the specific reaction without causing spontaneous agglutination of carrier particles are provided by using a certain kind of amine compound, and that the amine compound includes betaine hydrochloride. However, as can be seen from data shown in Examples of the document, when a comparison was made between a test sample of sensitized latex containing 150 mM betaine hydrochloride and a control sample without betaine hydrochloride, the test sample containing betaine showed better results only in two items out of three items measured with supernatants after leaving the reaction products at 4°C for 3 days, or only in one item measured after stirring the resultant. Thus, the data cannot be considered to show that the dispersed state of the sensitized latex was maintained by the addition of betaine and that sedimentation due to spontaneous agglutination and/or the like was prevented thereby. Moreover, the data do not show that agglutination due to freezing was prevented by the addition of betaine.

In a technical field different from that of immunoassay kits, it is known that addition of betaine to an inkjet ink composition prevents coagulation/sedimentation of the ink due to freeze-thawing (Patent Document 4). However, there is no known case of a latex reagent where betaine was added to a latex solution for the purpose of protection from deterioration due to freezing.

### PRIOR ART DOCUMENT(S)

### PATENT DOCUMENT(S)

Patent Document 1: JP H11-258241 A
Patent Document 2: JP S61-222531 A
Patent Document 3: WO 2007/074860
Patent Document 4: JP 2001-271013 A

### SUMMARY OF THE INVENTION

### PROBLEMS TO BE SOLVED BY THE INVENTION

An object of the present invention is to provide novel means for favorably maintaining the dispersed state of a latex reagent in an immunoassay kit and preventing occurrence of coagulation/sedimentation due to temperature changes so that the reagent is protected from deterioration in the measurement performance, which means is also applicable to an immunoagglutination assay kit containing a latex reagent aliquoted in small amounts.

### MEANS FOR SOLVING THE PROBLEMS

As substances that may be added to the latex reagent as an cryoprotective agent, sugars and surfactants have been known as described above. However, in cases of an immunoassay reagent for hemoglobin A1c using unsensitized latex, a sample prepared by diluting hemoglobin in a specimen is added to the latex reagent to allow physical adsorption of hemoglobin A1c onto the surface of the latex. In such an immunoassay reagent, a surfactant that inhibits the physical adsorption cannot be used as a cryoprotective agent. Moreover, a latex reagent which is aliquoted in very small amounts in cartridges needs to be allowed to flow freely in the small structure although the necessity depends on the structure. That is, if an additive increases the viscosity and decreases the fluidity of the latex reagent, the performance of the reagent may be affected, which is problematic. Therefore, cryoprotective agents with high molecular weights are not applicable to such a latex reagent.

In view of the above facts, the present inventors intensively studied to discover that addition of an appropriate amount of betaine (trimethylglycine) to a latex reagent enables prevention of coagulation/sedimentation of latex particles even after freeze-thawing, and allows the reagent to maintain its performance as an immunoassay reagent, thereby completing the present invention.

That is, the present invention provides an immunoassay reagent containing unsensitized latex particles and trimethylglycine in a solvent. The present invention also provides a method for preventing deterioration of an immunoassay reagent containing unsensitized latex particles, said method comprising allowing trimethylglycine to coexist in said immunoassay reagent.

### EFFECT OF THE INVENTION

By the present invention, a novel method for preventing deterioration in the performance of a latex reagent due to freeze-thawing was provided. The present invention prevents agglutination in a latex reagent even after freezing, and allows the reagent to maintain its performance as an immunoassay reagent. Thus, the reagent can be transported without causing deterioration in the performance even to an area where sufficient temperature control during the transportation is not available. Since betaine is a low-molecular-weight substance, it does not increase, unlike sugars, viscosity of the reagent even when it is used at a concentration of several percent. Thus, even when it is applied to a cartridge-type immunoassay kit containing the reagent in small aliquots, there is no concern of an adverse effect due to decreased fluidity. Moreover, unlike surfactants, there is no concern of preventing adsorption of the protein to be measured onto unsensitized latex particles.

### BRIEF DESCRIPTION OF THE DRAWINGS

Fig. 1 is a graph showing changes in the signal value observed when a latex reagent R1 supplemented with 6 w/v% betaine was subjected to 0 to 6 times of freeze-thawing treatment.
Fig. 2 shows calibration curves prepared by measuring signals from HbA1c standards using the latex reagent R1 supplemented with 6 w/v% betaine (freeze-thawing, 0 to 3 times). A calibration curve similarly prepared using the latex reagent R1 supplemented with a surfactant Tween 20 instead of betaine (freeze-thawing, 1 time) is also shown.

### MODE FOR CARRYING OUT THE INVENTION

The immunoassay reagent of the present invention is a reagent (latex reagent) in which latex particles are suspended in a solvent, and characterized in that unsensitized particles are used as the latex particles, and that the reagent contains betaine.

The term "unsensitized" means a state where a protein such as an antigen or an antibody is not substantially carried on the surface of latex particles. As an immunoassay method by latex agglutination, a method using unsensitized latex particles as the latex reagent is known (JP 2677753 B). In this method, unsensitized latex particles are brought into contact with a test sample to allow adsorption of the protein of interest onto the surface of the particles, and an antibody against the protein to be measured is then allowed to react with the particles to cause aggregation of the particles, followed by measuring the amount of the protein of interest based on the turbidity of the reaction liquid. The present invention can be suitably applied to immunoassays using such a method. In the present invention, the term "measurement" includes qualitative detection, quantification, and semi-quantification.

The latex particles *per se* may be the same as those used in known immunoagglutination assay kits. More specifically, the average particle size of the latex particles may be about 0.04 to 0.8 µm, for example, about 0.08 to 0.2 µm. The material of the latex is not limited, and a styrene latex such as a polystyrene latex; acrylic latex; or the like; is preferably used. It is preferred to use a latex whose surface has strong hydrophobicity (e.g., polystyrene latex) for smooth adsorption of the protein to be measured in the test sample. Various modified latices (e.g., carboxylate-modified latices), magnetic latices (latices internally containing magnetic particles), and the like may be used, if necessary.

The solvent may be a buffer conventionally used as a liquid in which latex is to be suspended for preparation of a latex reagent in an immunoassay kit based on latex agglutination. Examples of the solvent include, but are not limited to, glycine buffer, borate buffer, and Good's buffer. The concentration of latex in the reagent is usually about 0.05 to 0.5 w/v%.

The betaine used in the present invention is trimethylglycine (N,N,N-trimethylglycine). For allowing trimethylglycine to be contained in a solvent containing unsensitized latex, an anhydride, hydrate, or salt of trimethylglycine may be added to the solvent. Specific examples of the salt of trimethylglycine include, but are not limited to, hydrochloride, sulfate, and phosphate.

The content of betaine in the immunoassay reagent is preferably 5 to 30 w/v%, and may be, for example, 5 to 25 w/v%, 5 to 20 w/v%, 5 to 10 w/v%, 5.5 to 8.5 w/v%, or 5.5 to 7.5 w/v%. By incorporating betaine into the latex reagent, occurrence of coagulation/sedimentation of latex particles due to temperature changes (for example, freezing due to supercooling, and subsequent thawing) during transportation or storage of the reagent can be suppressed, and deterioration in the performance of the reagent as an immunoassay reagent can be prevented. At a betaine concentration within the range of 5 to 30 w/v%, there is no difference in the detected signal value between samples without freeze-thawing and samples subjected to single freeze-thawing, and an effect to prevent deterioration due to freezing can be obtained. At a betaine concentration of less than 5 w/v%, the detected signal value tends to increase due to repeated freeze-thawing, so that it is difficult to obtain an accurate measured value. On the other hand, at a betaine concentration of more than 8.5 w/v%, although a slight decrease in the detected signal value tends to occur due to repeated freeze-thawing, the detected signal value also decreases in samples without freeze-thawing, and it has been confirmed that the ratio of the decrease in the value depending on the number of times of freeze-thawing is almost the same, irrespective of the concentration. That is, at a concentration of more than 8.5 w/v%, the absolute value of the measured signal merely further decreased and the effect *per se* to prevent deterioration in the performance could be obtained; however, the effect to prevent deterioration did not further increase. Thus, the concentration of betaine used may be not more than 8.5 w/v%, for example, not more than 7.5 w/v%, although the concentration is not limited. However, since the optimal concentration of betaine to be contained in the latex reagent varies depending on the type and the concentration of the latex and the type of the buffer, the optimal betaine concentration may be investigated as appropriate. For example, since the detected signal value increases as the latex concentration in the reagent increases, the latex concentration may be high in cases where betaine is used at a high concentration.

Specific examples of the immunoassay utilizing an unsensitized latex reagent include immunoassays for hemoglobin A1c (HbA1c). Hemoglobin (Hb) has a heterotetramer structure consisting of two α-chains and two β-chains, and hemoglobin A1c (HbA1c) is a glycosylated hemoglobin generated by nonenzymatic glycosylation at the α-amino group of the N-terminal valine in each β-chain. In recent years, HbA1c is used as a criterion for diabetes. In cases where an immunoassay is carried out such that HbA1c is distinguished from non-glycosylated Hb, an antibody specific to the N-terminal region of the β-chain, which is a region specific to HbA1c, is employed. Although the N-terminal region of the β-chain is embedded in the higher order structure of the HbA1c molecule, the N-terminal region of the β-chain of HbA1c is exposed when HbA1c is adsorbed onto the surface of latex, so that an immunoassay using an anti-HbA1c antibody is possible without any special denaturation treatment. Commercially available HbA1c immunoassay kits using an unsensitized latex reagent are based on such a principle. The reagent of the present invention can be preferably applied to, for example, such HbA1c immunoassay kits.

The immunoassay for HbA1c by the agglutination method using the immunoassay reagent of the present invention can be carried out in the same manner as in a conventional method using the commercially available HbA1c immunoassay kit except that betaine is contained in an unsensitized latex reagent. More specifically, the process of the measurement is as follows. First, the reagent of the present invention is brought into contact with a test sample such as blood to allow adsorption of hemoglobin (which includes normal hemoglobin and glycosylated hemoglobin HbA1c) onto the surface of latex particles. Denaturation treatment of the sample is unnecessary, but pretreatment such as dilution hemolysis treatment or centrifugation may be carried out, if necessary. Subsequently, HbA1c adsorbed on the latex particles is brought into contact with an anti-HbA1c antibody. Since the anti-HbA1c antibody causes binding of the latex particles to one another via HbA1c to cause agglutination of the latex particles, the turbidity of the reaction liquid increases. By visual observation of the turbidity of the reaction liquid, the presence of HbA1c can be detected (qualitative detection). The turbidity may also be optically measured using an autoanalyzer or the like for quantification of the amount of HbA1c. In the latter case, standard samples with known HbA1c concentrations may be provided, and each standard sample may be subjected to optical measurement of the turbidity, followed by plotting the relationship between the measured values of turbidity and the concentrations of HbA1c, thereby preparing a calibration curve. By applying the measured value of the sample to this calibration curve, the amount of HbA1c in the sample can be determined.

Since the HbA1c adsorbed on the latex particles can also be used as a sample for an immunoassay together with the latex particles, the HbA1c on the latex particles may be measured by an immunoassay method other than the agglutination method. Methods of immunoassays *per se* are well known in the art. From the viewpoint of the reaction mode, immunoassays can be classified into the sandwich method, competition method, agglutination method, immunochromatography method, Western blotting method, and the like. From the viewpoint of the label, immunoassays can be classified into radioimmunoassay, fluorescence immunoassay, enzyme immunoassay (EIA), biotin immunoassay, and the like. All of these are included in the "immunoassay" in the present invention.

In cases where HbA1c is measured by the sandwich method, an anti-Hb antibody (which binds to total hemoglobin including HbA1c) may be immobilized on a solid phase, and a reaction liquid prepared by bringing the immunoassay reagent of the present invention into contact with a sample may be reacted with the immobilized antibody, followed by washing the resultant, reacting a labeled anti-HbA1c antibody therewith, washing the resultant, and then measuring the labeled antibody bound to the solid phase based on the signal from the label. The measurement may also be carried out by using an anti-HbA1c antibody as the immobilized antibody and using an anti-Hb antibody as the labeled antibody. Standard samples with known HbA1c concentrations are subjected to the measurement, and the relationship between the signal intensities and the HbA1c concentrations is plotted to prepare a calibration curve. By applying the measured value of a sample to this calibration curve, the amount of HbA1c in the sample can be quantified.

In cases where HbA1c is measured by lateral flow immunochromatography, which is an example of the sandwich method, the immunochromatography device may have the following constitution.

Usually, a matrix composed of a porous material such as a nitrocellulose membrane is formed into a belt shape. On this matrix, an anti-HbA1c monoclonal antibody is immobilized to provide a detection zone, and a labeled anti-Hb monoclonal antibody is spotted upstream thereof (upstream in the direction of the flow of the developer described later) to provide a labeled reagent zone. The labeled reagent zone is usually constituted by a porous pad on which a labeled antibody is spotted. At the upstream end of the matrix, a developer tank storing a developer is provided. In cases where an enzyme is used as the label, a substrate zone on which the substrate of the label enzyme is spotted may be provided upstream of the labeled reagent zone.

In the measurement, a reaction liquid obtained by allowing HbA1c to be adsorbed on the surface of the latex particles is added to the labeled reagent zone together with the latex particles contained therein. Thereafter, by breaking the developer tank, the developer is allowed to flow downstream by capillary action of the matrix. The developer sequentially passes through the substrate zone and the labeled reagent zone, and thus the substrate, the labeled antibody, and the latex particles on which hemoglobin is adsorbed flow downstream together with the developer. During this process, the hemoglobin on the surface of the latex particles binds to the labeled anti-Hb antibody due to antigen-antibody reaction. When the immune complex reaches the detection zone, the labeled antibody is captured by the anti-HbA1c antibody in the detection zone through HbA1c, which is contained in the hemoglobins on the surface of the latex particles. Since the substrate is flowing together, the detection zone, in which the labeled antibody is captured, is colored. This coloring may be visually observed to make a determination. Based on the presence/absence of the coloring, qualitative detection of HbA1c is possible. In addition, rough quantification is also possible based on the intensity of the coloring in the detection zone.

In addition to the above, an immunochromatography device in which an anti-Hb antibody is used as the antibody to be immobilized on the detection zone and an anti-HbA1c antibody is used as the labeled antibody to be spotted on the labeled reagent zone may also be used. An antibody against the labeled substance may be spotted next to the detection zone to provide a control zone in order to enable confirmation of appropriate developing of the labeled antibody.

Anti-HbA1c antibodies are known, and various HbA1c immunoassay kits using anti-HbA1c antibodies are commercially available. In the present invention, such known anti-HbA1c antibodies may be used. Since antibodies can be prepared by well-known conventional methods, an antibody which can distinguish HbA1c from non-glycosylated, normal hemoglobin to specifically recognize HbA1c may be prepared and used. As described above, the site specific to HbA1c is the N-terminal region of the β-chain. By immunizing an animal (excluding human) using the N-terminal region of the β-chain of HbA1c as an immunogen to induce antibodies against HbA1c in the body of the animal, polyclonal antibodies against HbA1c can be obtained from serum of the animal. In addition, spleen cells may be recovered from the immunized animal, and the conventional hybridoma method may be carried out to prepare an anti-HbA1c monoclonal antibody that specifically binds to HbA1c. From the viewpoint of reproducibility and the like in the immunoassay, an anti-HbA1c monoclonal antibody is preferably used.

Specific examples of the method for preparing the N-terminal region of the β-chain of HbA1c to be used as the immunogen and the method for preparing a monoclonal antibody against the region are described in detail in, for example, JP 2677753 B and the like, and those skilled in the art can appropriately prepare them. These processes are concretely described below.

The N-terminal region of the β-chain of HbA1c to be used as the immunogen can be prepared by synthesizing a peptide corresponding to the N-terminal region of the hemoglobin β-chain and nonenzymatically binding glucose to the α-amino group of the N-terminal amino acid residue, valine. The peptide to be subjected to the glycosylation treatment can be synthesized by a conventional method using a commercially available peptide synthesizer. The N-terminal region of the hemoglobin β-chain to be synthesized may be several residues in size. SEQ ID NO:1 shows the sequence of the 5 residues at the N-terminus of the human hemoglobin β-chain. The N-terminal peptide can be glycosylated by dissolving the prepared N-terminal peptide in acetic acid, adding twice the molar amount of glucose to the resulting solution in the presence of pyridine, and then stirring the resulting mixture at room temperature for about 10 days. Since glycosylation of the peptide reduces the retention time in HPLC, the progress of the glycosylation reaction can be checked by HPLC. The conditions of the analysis by HPLC may be, for example, as follows.

Column: TSKgel, ODS-120A (4.6 x 250 mm, manufactured by Tosoh Corporation)
Apparatus: Shimadzu HPLC System
Mobile phase: Linear gradient from 10% acetonitrile/0.1% trifluoroacetic acid to 60% acetonitrile/0.1% trifluoroacetic acid
Flow rate: 0.8 mL/min
Time: 25 min
Monitoring: Absorbance at 280 nm

The obtained glycosylated peptide (N-terminal region of the HbA1c β-chain) can be purified by HPLC under, for example, the following separation conditions.

Column: TSKgel, ODS-120A (21.5 x 300 mm, manufactured by Tosoh Corporation)
Mobile phase: Linear gradient from 10% acetonitrile/0.1 % trifluoroacetic acid to 60% acetonitrile/0.1% trifluoroacetic acid
Flow rate: 5 mL/min
Monitoring: Absorbance at 280 nm

From the purified peptide, the cysteine protecting group added during the chemical peptide synthesis process may be eliminated by a conventional method, and HPLC purification may be carried out again under the same separation conditions as described above (except that the absorbance for monitoring may be 215 nm), to provide an immunogen.

Since the N-terminal region of the HbA1c β-chain produced as described above is several residues in size, when the peptide is used as an immunogen, the peptide may be bound to an appropriate carrier protein (e.g., thyroglobulin, edestin or the like) and mixed with an appropriate adjuvant (e.g., complete Freund adjuvant, incomplete Freund adjuvant or the like). The resulting mixture may then be used for immunization of an animal (excluding human). By this, as described above, antibodies against the immunogen can be induced in the body of the animal, and spleen cells may then be recovered from the animal, followed by preparing a monoclonal antibody by the hybridoma method.

Screening of an anti-HbA1c antibody having a desirable specificity to HbA1c can be carried out by, for example, providing a plate on which normal hemoglobin is immobilized and a plate on which HbA1c is immobilized, and investigating reactivity of a plurality of lines of obtained antibodies with both plates. An antibody whose reactivity with the normal hemoglobin is about not more than that of the background and whose reactivity with HbA1c is high can be preferably used as an HbA1c-specific antibody.

The method for using the immunoassay reagent of the present invention is described above by way of an example in which HbA1c is measured, but the immunoassay reagent of the present invention can be used for measurement of any substance as long as the substance can be measured by an immunoassay using unsensitized latex particles.

### EXAMPLES

The present invention is described below concretely by way of Examples. However, the present invention is not limited to the Examples below.

### <Study 1>

Each of an unsensitized latex reagent (hereinafter referred to as R1 reagent) and an anti-HbA1c antibody solution (hereinafter referred to as R2 reagent) contained in a known HbA1c immunoassay kit was dispensed in an amount of about 5 mL into a 10-mL tube. Tubes were then stored in a freezer at -20°C overnight to allow freezing of the R1 reagent and the R2 reagent. Subsequently, the reagents were left to stand at room temperature to allow thawing, and the conditions of each reagent were visually observed.

In the R1 reagent, coagulation/sedimentation of latex occurred to produce a white precipitate, and the solution after the precipitation was transparent. Thus, the reagent was apparently unusable as an immunoassay reagent. On the other hand, no apparent change was found in the R2 reagent. In view of this, the R2 reagent that had not undergone freeze-thawing and the R2 reagent that underwent freeze-thawing once were placed in a Hitachi 7170 autoanalyzer together with the R1 reagent that had not undergone freeze-thawing, and calibration curves were prepared by subjecting HbA1c standard samples to the measurement. The resulting calibration curves were identical. It could therefore be confirmed that the measurement performance of the antibody solution was not affected by freeze-thawing. Thus, the study of protection from deterioration due to freezing was carried out for the latex reagent.

### <Study 2>

To the R1 reagent, betaine (trimethylglycine) was added at concentrations within the range of 3 w/v% to 10 w/v% at intervals of 1%, and the influence of freeze-thawing was investigated. The R1 reagent supplemented with betaine at each concentration was divided into 3 equal portions, and one of these portions was stored at 4°C. In the same manner as in Study 1, the other two portions were stored in a freezer at -20°C overnight, and, on the next day, left to stand at room temperature to allow thawing. As a result, no coagulation/sedimentation of latex was found, but the concentration of latex, which appeared white, was high at the bottom of each tube. The reagent samples were therefore gently mixed by inversion of the tubes. Subsequently, one of the two tubes was stored at 4°C, and the other tube was stored at -20°C overnight. On the next day, the reagent sample was thawed at room temperature, and gently mixed by inversion.

The R1 reagent samples that had undergone 0, 1, or 2 times of freeze-thawing were placed in a Hitachi 7170 autoanalyzer, and calibration curves were prepared by subjecting standard HbA1c samples to measurement at a secondary wavelength of 800 nm/primary wavelength of 660 nm. Table 1 shows 2-point-end signals of the standard samples.

**[Table 1]**

| Betaine concentration | Number of times of freeze-thawing | Signal (2 point end) | | | | |
|---|---|---|---|---|---|---|
| | | STD 0 | STD L | STD M1 | STD M2 | STD H |
| 3 w/v% | 0 time | -9 | 135 | 546 | 1357 | 2290 |
| | 1 time | 21 | 377 | 1148 | 2316 | 3304 |
| | 2 times | 23 | 409 | 1214 | 2325 | 3223 |
| 4 w/v% | 0 time | -10 | 131 | 511 | 1246 | 2096 |
| | 1 time | 9 | 182 | 633 | 1474 | 2458 |
| | 2 times | 12 | 197 | 677 | 1548 | 2553 |
| 5 w/v% | 0 time | -10 | 123 | 478 | 1161 | 1959 |
| | 1 time | -4 | 126 | 467 | 1140 | 1957 |
| | 2 times | 2 | 140 | 511 | 1218 | 2046 |
| 6 w/v% | 0 time | -7 | 119 | 459 | 1115 | 1865 |
| | 1 time | -10 | 118 | 450 | 1080 | 1840 |
| | 2 times | -5 | 124 | 463 | 1101 | 1872 |
| 7 w/v% | 0 time | -11 | 115 | 436 | 1030 | 1734 |
| | 1 time | -6 | 114 | 433 | 1026 | 1735 |
| | 2 times | -6 | 108 | 407 | 980 | 1687 |
| 8 w/v% | 0 time | -7 | 110 | 404 | 953 | 1608 |
| | 1 time | -10 | 108 | 401 | 950 | 1611 |
| | 2 times | -6 | 105 | 387 | 929 | 1577 |
| 9 w/v% | 0 time | -9 | 103 | 381 | 885 | 1487 |
| | 1 time | -8 | 105 | 380 | 901 | 1496 |
| | 2 times | -9 | 98 | 361 | 851 | 1454 |
| 10 w/v% | 0 time | -8 | 97 | 356 | 825 | 1381 |
| | 1 time | -11 | 98 | 356 | 825 | 1391 |
| | 2 times | -8 | 93 | 339 | 796 | 1358 |

It could be confirmed that the signal value was stable even after repeating freeze-thawing at a betaine concentration of around 6 w/v%. At lower betaine concentrations, the signal tended to increase due to repeating of freeze-thawing, whereas, at higher betaine concentrations, the signal tended to decrease due to repeating of freeze-thawing.

### <Study 3>

Based on the results of Study 2, it was thought that the appropriate concentration of betaine in the R1 reagent was about 6w/v%. In view of this, R1 reagent samples supplemented with betaine at a concentration of 6 w/v% or 7 w/v% were prepared, and each reagent sample was divided into 7 portions. One of these portions was stored at 4°C, and the other 6 portions were stored at -20°C overnight. On the next day, all portions were thawed at room temperature, and gently mixed by inversion. Subsequently, one of the portions was stored at 4°C, and the other portions were stored at -20°C overnight. This process was repeated to provide R1 reagent samples which had undergone 0 to 6 times of freeze-thawing. These R1 reagent samples and the R2 reagent were placed in a Hitachi 7170 autoanalyzer to prepare calibration curves.

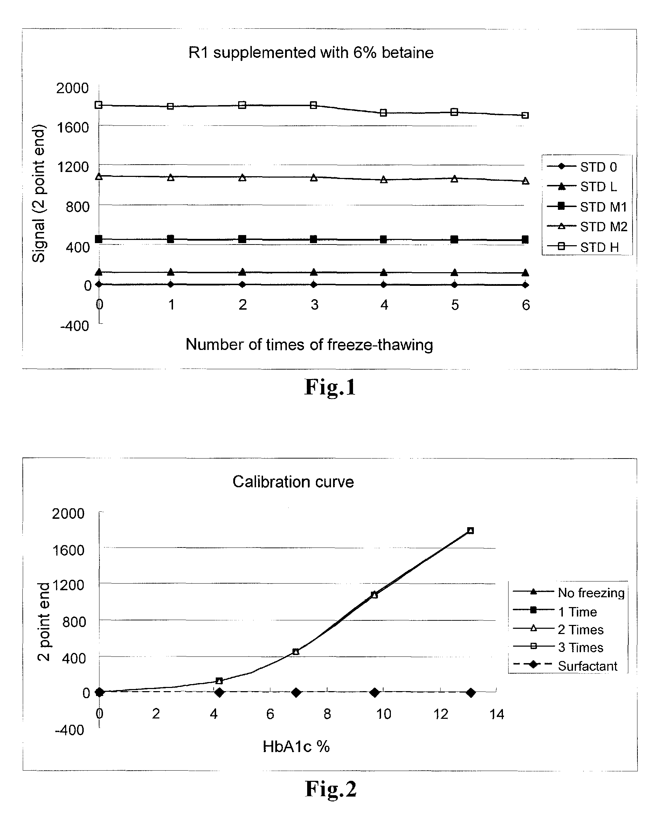

Fig. 1 shows changes in the signals in the calibration curves observed when latex reagent R1 supplemented with 6 w/v% betaine was subjected to freeze-thawing up to 6 times. No significant change was observed in the case where freeze-thawing was carried out up to 3 times, whereas, decreases in the signal were observed in the case where freeze-thawing was carried out 4 or more times. The same results were observed also in the case of 7 w/v% betaine.

Fig. 2 shows a superimposed image of the calibration curves prepared by using latex reagent R1 which was supplemented with 6 w/v% betaine and had undergone freeze-thawing up to 3 times. Since there were hardly changes observed in the signal, it was revealed that, even in cases where the latex reagent is frozen, the reagent can still react after thawing, similarly to the R1 reagent that has not undergone freeze-thawing. That is, it can be said that the latex reagent still satisfies the stability strictly required for the reagent. Similar results were obtained also for the R1 reagent samples supplemented with 7 w/v% betaine.

### (Study 4)

To an unsensitized latex reagent equivalent (R1 reagent) contained in a known HbA1c immunoassay kit, betaine was added at 5% to 25%. Since the latex concentration decreased by the addition of betaine, the concentration was adjusted by addition of a stock solution of latex. Part of the unsensitized latex reagent supplemented with betaine was left to stand at -20°C for 2 overnights, and then left to stand at room temperature to allow thawing. Thereafter, the R1 reagent samples after mixing by inversion and the refrigerated R1 reagent were placed in a Hitachi 7170 autoanalyzer, and calibration curves were prepared. An anti-HbA1c antibody solution (R2 reagent) containing a slightly larger amount of a sensitizer was used.

Table 2 shows 2-point-end signals of the standard samples at a secondary wavelength of 800 nm/primary wavelength of 660 nm. There was no difference observed between R1 reagent samples with freezing and R1 reagent samples without freezing. Calibration curves were prepared and measurement of controls was carried out. As a result, the measured values were found to be constant.

**[Table 2]**

| Addition of betaine | 5% | | 10% | | 15% | | 20% | | 25% | |
|---|---|---|---|---|---|---|---|---|---|---|
| Freezing | No | Yes | No | Yes | No | Yes | No | Yes | No | Yes |
| STD (A1c%) | | | | | | | | | | |
| 0 | -8 | -13 | -12 | -14 | -14 | -16 | -16 | -18 | -19 | -21 |
| 4.2 | 214 | 219 | 176 | 176 | 142 | 143 | 116 | 117 | 87 | 84 |
| 6.9 | 801 | 807 | 624 | 618 | 480 | 488 | 369 | 375 | 274 | 266 |
| 9.7 | 1941 | 1961 | 1446 | 1451 | 1081 | 1085 | 813 | 807 | 583 | 568 |
| 13.3 | 3385 | 3417 | 2521 | 2524 | 1869 | 1878 | 1380 | 1396 | 985 | 966 |
| Measured value | | | | | | | | | | |
| CTL Low | 6.0 | 6.0 | 6.0 | 6.0 | 6.0 | 6.0 | 6.0 | 6.0 | 6.0 | 6.0 |
| CTL High | 10.0 | 10.1 | 10.1 | 10.1 | 10.1 | 10.1 | 10.1 | 10.2 | 10.1 | 10.3 |

Table 3 shows results obtained in the same manner as in the study for Table 2 except that the latex concentration in the R1 reagent was increased by 50%. By the increase in the latex concentration, the 2-point-end-signals of the standard products were largely increased, and no difference was found between reagent samples with freezing and reagent samples without freezing. The calibration curve was not prepared using the reagent sample supplemented with 5% betaine, since the ABS exceeded a predetermined value. The measured values for controls were constant according to calibration curves prepared.

**[Table 3]**

| Addition of betaine | 5% | | 10% | | 15% | | 20% | | 25% | |
|---|---|---|---|---|---|---|---|---|---|---|
| Freezing | No | Yes | No | Yes | No | Yes | No | Yes | No | Yes |
| STD (A1c%) | | | | | | | | | | |
| 0 | -31 | -29 | -33 | -38 | -33 | -37 | -38 | -42 | -45 | -47 |
| 4.2 | 459 | 464 | 369 | 369 | 302 | 299 | 238 | 231 | 183 | 178 |
| 6.9 | 2004 | 2030 | 1454 | 1454 | 1084 | 1079 | 799 | 778 | 573 | 565 |
| 9.7 | 5406 | 5431 | 3822 | 3785 | 2676 | 2654 | 1850 | 1802 | 1257 | 1248 |
| 13.3 | 7918 | 8047 | 6101 | 6197 | 4485 | 4462 | 3162 | 3092 | 2136 | 2093 |
| Measured value | | | | | | | | | | |
| CTL Low | | | 6.0 | 6.0 | 6.0 | 5.9 | 6.0 | 6.0 | 5.9 | 6.0 |
| CTL High | | | 10.0 | 10.1 | 10.1 | 10.0 | 10.1 | 10.1 | 10.1 | 10.1 |

### (Comparative Example)

A reagent sample was prepared by adding a surfactant Tween 20, instead of betaine, at 0.1 v/v% to the latex reagent R1. The R1 reagent supplemented with the surfactant was placed in a tube, and the tube was then stored at -20°C overnight, followed by thawing the reagent on the next day at room temperature. Based on visual observation, no coagulation/sedimentation of latex was found. A calibration curve was then prepared with the R1 reagent supplemented with the surfactant using Hitachi 7170. The results are shown in Fig. 2, together with the results of Study 3. Since no reactivity could be observed, it is clear that the surfactant prevented physical adsorption of HbA1c onto the surface of the latex.

## Claims

1. An immunoassay reagent comprising unsensitized latex particles and trimethylglycine in a solvent.

2. The reagent according to claim 1, wherein the trimethylglycine concentration in said reagent is 5 to 30 w/v%.

3. The reagent according to claim 1, wherein the trimethylglycine concentration in said reagent is 5 to 10 w/v%.

4. The reagent according to any one of claims 1 to 3, which is an immunoassay reagent for an agglutination method.

5. The reagent according to any one of claims 1 to 4, which is an immunoassay reagent for hemoglobin A1c.

6. A method for preventing deterioration of an immunoassay reagent containing unsensitized latex particles, said method comprising allowing trimethylglycine to coexist in said immunoassay reagent.

7. The method according to claim 6, wherein the trimethylglycine concentration in said reagent is 5 to 30 w/v%.

8. The method according to claim 6, wherein the trimethylglycine concentration in said reagent is 5 to 10 w/v%.

9. The method according to any one of claims 6 to 8, wherein said immunoassay reagent is an immunoassay reagent for an agglutination method.

10. The method according to any one of claims 6 to 9, wherein said immunoassay is an immunoassay reagent for hemoglobin A1c.
